# FASCICULE DE BREVET EUROPEEN

(11) **EP 2 868 329 B1**
(45) Date de publication et mention de la délivrance du brevet: **10.10.2018**
(21) Numéro de dépôt: 14190555.4
(22) Date de dépôt: 27.10.2014
(51) Int. Cl.: A61L 2/14, B08B 9/28, H01T 23/00, B08B 5/02, B08B 6/00

(54) **Dispositif de nettoyage de flacons**
Reinigungsvorrichtung für Flaschen
Device for cleaning bottles

(30) Priorité: 30.10.2013 FR 1360649
(43) Date de publication de la demande: 06.05.2015
(73) Titulaire: ELCOWA, 68200 Mulhouse (FR)
(72) Inventeur: Lebon, Henri, 68270 WITTENHEIM (FR)
(74) Mandataire: Hege, Frédéric

(56) Documents cités:
- DE-A1- 10 140 906
- NL-A- 9 001 225

## Description

L'invention concerne le domaine de l'industrie cosmétique, pharmaceutique, alimentaire ou de verrerie, et concerne plus particulièrement un dispositif de nettoyage de l'intérieur de flacons ou tubes.

Dans le cadre du remplissage de flacons ou tubes, les parois internes doivent être garanties propres, avant leur remplissage par un produit. Or dans le cas de flacons ou tubes à goulot ou ouverture de faible diamètre, ceci n'est pas aisé à réaliser.

On connaît des dispositifs d'ionisation, permettant de décoller les poussières, associés à un dispositif de soufflage, assurant l'élimination de ces poussières. La création d'ions positifs et négatifs grâce à l'application d'une tension alternative à une pointe permet d'éliminer les charges sur les parois, charges qui ont tendance à retenir les poussières. Les poussières se séparent alors des parois, et un jet d'air permet de les emporter.

Le document DE 101 40 906 A1 décrit un dispositif de nettoyage de préformes en plastique comportant une tige reliée à un raccord d'air munie d'une pointe à son extrémité dans laquelle vient se loger une électrode d'ionisation. Toutefois un tel procédé ne peut pas être appliqué à l'intérieur d'un flacon si le goulot est trop petit. On peut alors introduire une pointe ionisante dans le flacon, et tenter tant bien que mal d'insuffler de l'air comprimé par le goulot, en espérant que celui-ci emporte le plus de poussière possible avant de ressortir par le même goulot. Or l'air a du mal à ressortir par le goulot par lequel l'air comprimé est introduit au même moment, et le nettoyage est donc largement imparfait à partiel.

La présente invention se propose de remédier à au moins une partie des inconvénients précités et propose une solution qui permette d'ioniser et de nettoyer efficacement l'intérieur d'un flacon à goulot de faible diamètre.

A cet effet, l'invention concerne un dispositif d'ionisation et de soufflage comportant une tige conductrice munie d'une pointe à son extrémité et reliée électriquement à une prise électrique de sorte à pouvoir appliquer une tension à ladite pointe, et une conduite d'air reliée à un raccord d'air de sorte à pouvoir souffler de l'air par ladite conduite d'air. Ce dispositif est particulier en ce que ladite tige est creuse, formant un canal constituant ladite conduite d'air.

Une telle disposition permet de faire entrer de l'air à travers la tige conductrice, ce qui permet de laisser libre le reste du goulot pour la sortie de l'air introduit dans un flacon. Selon d'autres caractéristiques :
- ladite pointe peut être disposée à l'extrémité de ladite tige en dehors de la section du canal, de sorte qu'une partie substantielle de l'air soufflé en extrémité du canal peut poursuivre sa course dans le sens de la tige ; une telle disposition permet d'améliorer très nettement l'effet de soufflage sur le fond du flacon, alors que si les pointes sont disposées dans la section du canal, l'air est dévié par les côtés et a du mal à atteindre le fond du flacon,
- ladite tige peut être circulaire, et comporter deux pointes disposées diamétralement opposées en extrémité de la tige, au niveau des parois de ladite tige ; une telle disposition améliore l'efficacité de l'ionisation par le doublement des pointes, sans occuper d'espace pouvant gêner le flux d'air, ou produisant l'effet d'augmenter la dimension de la partie du dispositif à introduire dans le flacon,
- l'extrémité de ladite tige peut présenter une forme de deux faces planes sécantes selon une arête perpendiculaire à l'axe de la tige, et les deux pointes être disposées sensiblement sur ladite arête au niveau des parois de ladite tige ; une telle disposition permet une fabrication aisée de la pointe,
- les deux pointes peuvent être disposées au niveau de l'intérieur des parois de ladite tige, permettant là encore un taillage facile des pointes, par un outil approchant par l'extérieur de la tige,
- les deux pointes peuvent être disposées au niveau de l'extérieur des parois de ladite tige, permettant une distance maximale entre les deux pointes, pour maximiser l'effet d'ionisation.

L'avantage découlant de la présente invention consiste en ce que l'effet de soufflage est très nettement amélioré, l'air entrant dans le flacon ne gênant plus l'air sortant. Selon des modes de réalisation avantageux, on peut obtenir un soufflage particulièrement efficace sur le fond du flacon, une efficacité améliorée de l'ionisation par les deux pointes, ou d'autres avantages encore.

D'autres caractéristiques et avantages de l'invention ressortiront de la description détaillée qui va suivre se rapportant à un exemple de réalisation donné à titre indicatif et non limitatif.

La compréhension de cette description sera facilitée en se référant aux dessins joints, représentant un exemple de réalisation, dans lesquels :
- la figure 1 représente une vue en coupe longitudinale d'un dispositif selon l'invention,
- la figure 2 représente une vue détaillée de face de l'extrémité de la tige du dispositif de la figure 1 ;
- la figure 3 représente une vue de côté de la pointe de la fig. 2 ;
- la figure 4 représente le détail d'une pointe selon un autre mode de réalisation.

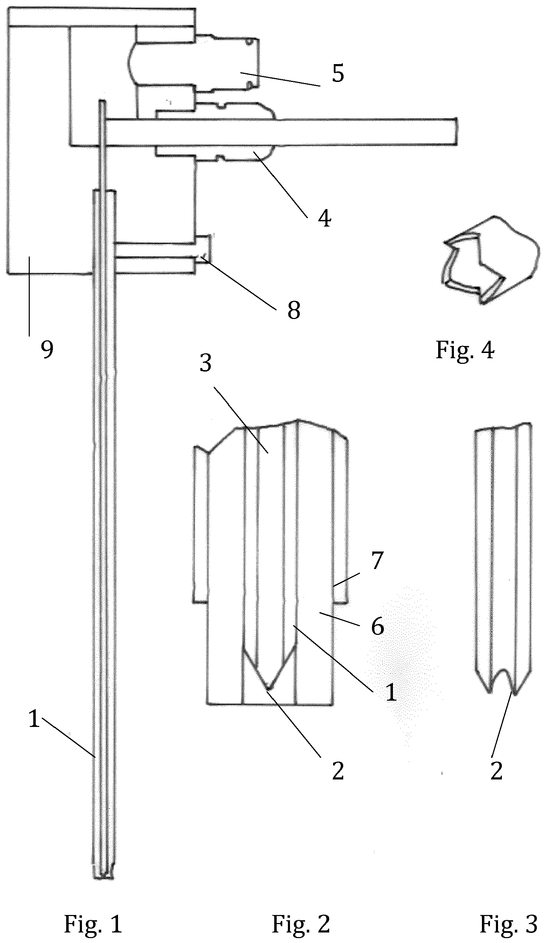

Tel que représenté dans les figures 1 et 2 du dessin ci-joint, la présente invention concerne un dispositif d'ionisation et de soufflage adapté à nettoyer efficacement le fond d'un flacon à goulot de faible diamètre.

Ce dispositif comporte une tige 1 conductrice, munie en l'une de ses extrémités d'au moins une, et de préférence deux pointes 2. Cette tige 1 est raccordée à une prise électrique 4 à haute tension, typiquement 5kV alternatif de sorte à pouvoir conférer une telle tension à la tige 1, en particulier au niveau de la pointe 2. La tige 1 est creuse, un canal 3 y est disposé à l'intérieur, apte à laisser passer de l'air. Le canal 3 est relié à un raccord d'air 5, de sorte à pouvoir souffler de l'air, qui débouche du canal 3 au niveau de la pointe 2. Le canal 3 est débouchant en son extrémité de sorte que l'air débouchant poursuit son mouvement dans le sens du canal 3, et est donc propulsé directement sur le fond du flacon. Cette disposition permet par ailleurs de créer un jet d'air punctiforme, plus efficace qu'un jet dispersé. D'autres dispositions des pointes 2 peuvent convenir également, dans lesquelles les pointes 2 peuvent par exemple être disposées dans l'encombrement du canal 3, plus proches l'une de l'autre, et l'air débouchant être au moins en partie propulsé latéralement. Toutefois le fond du flacon est alors bien moins bien nettoyé, quoique encore significativement mieux qu'avec un soufflage d'air non pas par l'intérieur de la tige 1, mais directement par le goulot.

Les pointes 2 sont obtenues par deux usinages plans de l'extrémité de la tige 1, selon deux plans se rejoignant selon une droite perpendiculaire à la tige 1 et passant par l'axe de la tige 1. Si la tige 1 était pleine, cela donnerait une arête perpendiculaire à l'axe de la tige 1, mais du fait du canal 3 débouchant, cela donne deux petites arêtes, correspondant à l'intersection de ladite arête avec les deux parois de la tige 1. Il suffit alors de tailler ces deux arêtes en pointes 2, avec un choix possible de l'éloignement entre les deux pointes 2 entre un diamètre de canal 3 au minimum, et un diamètre extérieur de la tige 1 au maximum, cet éloignement étant choisi selon l'intensité de l'effet d'ionisation recherché. On peut alors choisir de disposer les pointes 2 au niveau de la face interne de la tige 1 creuse, comme représenté à la fig. 3, ou alors au niveau de sa face externe, ou en n'importe quelle position entre ces deux positions.

Selon les applications, le diamètre de la tige peut varier par exemple entre 0,5 et 10mm. Dans les cas de diamètres plus importants, on peut plus facilement tailler un plus grand nombre de pointes, comme illustré à la fig. 4, montrant quatre pointes.

La tige 1 est recouverte d'une couche 6 isolante, par exemple en PTFE (polytétrafluoroethylène), communément appelé Teflon® (marque déposée), lequel est entouré d'une enveloppe 7 conductrice, par exemple en inox, reliée à la terre. Une prise de mise à la terre 8 est prévue sur le dispositif, reliée électriquement à ladite enveloppe 7.

Un boîtier 9 permet de regrouper, de maintenir en place et de protéger les divers raccordements. Le dispositif se présente ainsi avec ledit boîtier 9 à une extrémité, et la tige 1 dépassant du boîtier 9, seule l'enveloppe 7 étant visible, sauf en extrémité de tige 1, où la pointe 2 est visible en vue en bout, bien entourée par la couche 6 isolante.

La tige 1 peut présenter par exemple une longueur d'environ 100, 160 ou 250mm, cette longueur étant bien entendu à adapter à la profondeur des flacons à nettoyer. Le diamètre du goulot d'un flacon, ou de l'ouverture d'un tube peut être réduit à seulement quelques millimètres, le dispositif étant adapté à nettoyer l'intérieur de tels flacons ou tubes jusqu'à une dimension de goulot ou ouverture de 20, voire 40mm. Au-delà le dispositif fonctionne bien entendu également, mais d'autres dispositifs de l'état de la technique peuvent être implantés quand l'ouverture devient suffisamment grande, et l'avantage du dispositif selon l'invention s'atténue.

Le dispositif selon l'invention peut être installé sur une chaine de remplissage de flacons de cosmétique, juste avant l'opération de remplissage proprement dit, par exemple pour nettoyer l'intérieur des flacons de parfum, shampoing, gel douche, stick rouge à lèvres. Dans le domaine pharmaceutique il peut s'agir de flacons de sirop, de tubes à essais, d'étuis à granulés ou comprimés. Dans le domaine alimentaire il peut s'agir de bouteilles. Le dispositif selon l'invention peut aussi être implanté pour le nettoyage en sortie de ligne de fabrication de flacons ou de bouteilles de verre dans l'industrie verrière.

Le boîtier 9 est alors disposé au-dessus du goulot du flacon. On applique alors la haute tension au niveau de la prise électrique 4, et la tige 1 est introduite dans le flacon jusqu'à ce que la pointe 2 se trouve proche du fond du flacon. L'homme du métier saura parfaitement déterminer la longueur nécessaire de la tige 1, ainsi que la distance à maintenir entre la pointe 2 et le fond du flacon pour obtenir le meilleur nettoyage.

On démarre alors le soufflage. La pointe 2 produit son effet sur le fond et les parois internes du flacon, et permet de décoller les poussières. Le flux d'air sortant du canal 3 au niveau de la pointe 2 lèche alors les parois internes et s'évacue par le goulot, emportant la poussière vers l'extérieur du flacon. En effet, rien n'empêche l'air de sortir par le goulot, puisqu'on ne cherche pas à y faire entrer de l'air en même temps. On peut aussi ajouter un dispositif d'aspiration d'air au niveau du goulot, pour faciliter encore la sortie de l'air chargé des poussières. On peut alors retirer la tige jusqu'à sortir du goulot avant d'arrêter la tension et l'air comprimé. L'homme du métier saura adapter ce procédé aux conditions d'utilisation, par exemple en changeant l'ordre des différentes étapes selon ses besoins spécifiques.

L'avantage de la présente invention réside en particulier en ce que le dispositif selon l'invention permet d'obtenir un meilleur nettoyage de l'intérieur d'un flacon à goulot étroit, et en particulier de son fond, tout en réduisant la consommation d'air. Selon un mode avantageux de réalisation de l'invention, la présence de deux pointes 2 judicieusement éloignées l'une de l'autre permet d'augmenter l'intensité d'ionisation pour améliorer encore le nettoyage ou pour permettre une réduction de la consommation d'électricité.

### Nomenclature

- 1.: tige
- 2.: pointe
- 3.: canal
- 4.: prise électrique
- 5.: raccord d'air
- 6.: couche isolante
- 7.: enveloppe
- 8.: prise de terre
- 9.: boîtier

## Revendications

1. Dispositif d'ionisation et de soufflage comportant une tige (1) conductrice munie d'une pointe (2) à son extrémité et reliée électriquement à une prise électrique (4) de sorte à pouvoir appliquer une tension à ladite pointe (2), et une conduite d'air reliée à un raccord d'air (5) de sorte à pouvoir souffler de l'air par ladite conduite d'air, **caractérisé en ce que** ladite tige (1) est creuse, formant un canal (3) constituant ladite conduite d'air.

2. Dispositif selon la revendication précédente, dans lequel ladite pointe (2) est disposée à l'extrémité de ladite tige (1) en dehors de la section du canal (3), de sorte qu'une partie substantielle de l'air soufflé en extrémité du canal (3) peut poursuivre sa course dans le sens de la tige (1).

3. Dispositif selon la revendication précédente, dans lequel ladite tige (1) est circulaire, et comporte deux pointes (2) disposées diamétralement opposées en extrémité de la tige (1), au niveau des parois de ladite tige (1).

4. Dispositif selon la revendication précédente dans lequel l'extrémité de ladite tige (1) présente une forme de deux faces planes sécantes selon une arête perpendiculaire à l'axe de la tige (1), et les deux pointes (2) sont disposées sensiblement sur ladite arête au niveau des parois de ladite tige (1).

5. Dispositif selon la revendication précédente, dans lequel les deux pointes (2) sont disposées au niveau de l'intérieur des parois de ladite tige (1).

6. Dispositif selon la revendication 4, dans lequel les deux pointes (2) sont disposées au niveau de l'extérieur des parois de ladite tige (1).

7. Procédé de nettoyage d'un flacon ou d'un tube par le moyen d'un dispositif selon l'une des revendications précédentes, et comportant les étapes suivantes :
- mise sous tension de la prise électrique (4),
- introduction de la tige (1) dans le flacon ou le tube,
- application d'air comprimé au niveau du raccord d'air (5),
- retrait de la tige (1) jusqu'à sortir du flacon ou du tube,
- arrêt tension et air comprimé.

## Patentansprüche

1. Ionisations- und Blasvorrichtung mit einem leitenden Stab (1), der an seinem Ende mit einer Spitze (2) versehen und elektrisch mit einer elektrischen Buchse (4) verbunden ist, um eine Spannung an die Spitze (2) anlegen zu können, und einem Luftkanal, der mit einem Luftanschluss (5) verbunden ist, um Luft durch den Luftkanal blasen zu können, **dadurch gekennzeichnet, dass** der Stab (1) hohl ist und einen Kanal (3) bildet, der den Luftkanal bildet.

2. Vorrichtung nach dem vorstehenden Anspruch, wobei die Spitze (2) am Ende des Stabs (1) außerhalb des Kanalabschnitts (3) angeordnet ist, so dass ein wesentlicher Teil der am Kanalende (3) geblasenen Luft ihren Weg in Richtung des Stabs (1) fortsetzen kann.

3. Vorrichtung nach dem vorstehenden Anspruch, bei der der Stab (1) kreisförmig ist und zwei diametral gegenüberliegende Spitzen (2) am Ende des Stabs (1) in Höhe der Wände des Stabs (1) aufweist.

4. Vorrichtung nach dem vorstehenden Anspruch, bei der das Ende des Stabs (1) eine Form von zwei sich kreuzenden flachen Flächen entlang einer Kante senkrecht zur Achse des Stabs (1) aufweist und die beiden Spitzen (2) im wesentlichen auf der Kante in Höhe der Wände des Stabs (1) angeordnet sind.

5. Vorrichtung nach dem vorstehenden Anspruch, bei der die beiden Spitzen (2) auf der Innenseite der Wände des Stabs (1) angeordnet sind.

6. Vorrichtung nach Anspruch 4, wobei die beiden Spitzen (2) an der Außenseite der Wände des Stabs (1) angeordnet sind.

7. Verfahren zum Reinigen einer Flasche oder einem Rohr mittels einer Vorrichtung nach einem der vorstehenden Ansprüche, umfassend die folgenden Schritte :
- Einschalten der Steckdose (4),
- Einführen des Stabs (1) in die Flasche oder das Rohr,
- Anwendung von Druckluft am Luftanschluss (5),
- Entfernung des Stabs (1), bis er aus der Flasche oder dem Rohr ist,
- Spannungs- und Druckluftabschaltung.

## Claims

1. Device for ionization and blowing comprising a conductive rod (1) provided with a tip (2) at its end and electrically connected to an electrical socket (4) so as to be able to apply a voltage to said tip (2), and an air duct connected to an air connection (5) so as to be able to blow air through said air duct, **characterized in that** said rod (1) is hollow, forming a channel (3) constituting said air duct.

2. Device as claimed above, wherein said tip (2) is arranged at the end of said rod (1) outside the channel (3) section, so that a substantial portion of the air blown at the channel (3) end can continue its course in the direction of the rod (1).

3. Device according to the previous claim, wherein said rod (1) is circular, and comprises two tips (2) arranged diametrically opposed at the end of the rod (1), at the level of the walls of said rod (1).

4. Device according to the previous claim wherein the end of said rod (1) has a shape of two intersecting flat faces along an edge perpendicular to the axis of the rod (1), and the two tips (2) are arranged substantially on said edge at the level of the walls of said rod (1).

5. Device according to the previous claim, wherein the two tips (2) are arranged on the inside of the walls of said rod (1).

6. Device according to claim 4, wherein the two tips (2) are arranged at the outside of the walls of said rod (1).

7. Method for cleaning a bottle or tube by means of a device according to one of the previous claims, and comprising the following steps :
- switching on the electrical socket (4),
- introduction of the rod (1) into the bottle or tube,
- application of compressed air at the air connection (5),
- removal of the rod (1) until it comes out of the bottle or tube,
- shutting down the voltage and the compressed air.
